# EUROPEAN PATENT SPECIFICATION

(11) **EP 1 126 867 B1**
(45) Date of publication and mention of the grant of the patent: **14.05.2003**
(21) Application number: 99970026.3
(22) Date of filing: 30.09.1999
(51) Int. Cl.: A61K 38/17, A61P 9/10, A61P 37/00

(54) **A COMPOSITION FOR THE PREVENTION AND/OR TREATMENT OF ATHEROSCLEROSIS**
ZUSAMMENSETZUNG ZUR VORBEUGUNG UND/ODER BEHANDLUNG DER ATHEROSKLEROSE
COMPOSITION DESTINEE A LA PREVENTION ET/OU AU TRAITEMENT DE L'ATHEROSCLEROSE

(30) Priority: 04.10.1998 IL 12644798
(43) Date of publication of application: 29.08.2001
(73) Proprietor: Vascular Biogenics Ltd., 60376 Or Yehuda (IL)
(72) Inventor: SHOENFELD, Yehuda, 52622 Ramat Gan (IL); HARATS, Dror, 52623 Ramat Gan (IL); GEORGE, Jacob, 49311 Petach-Tiqva (IL)
(74) Representative: Schmitz, Jean-Marie
(86) International application number: IL9900519
(87) International publication number: WO00020019

(56) References cited:
- US-A- 5 348 945
- GEORGE JACOB ET AL: "Induction of early atherosclerosis in LDL-receptor-deficient mice immunized with beta2-glycoprotein I." CIRCULATION SEPT. 15, 1998, vol. 98, no. 11, pages 1108-1115, XP000892137 ISSN: 0009-7322
- DATABASE BIOSIS [Online] BIOSCIENCES INFORMATION SERVICE, PHILADELPHIA, PA, US; 1998 BONNIN DUSTAN ET AL: "Mucosal modulation of immune responses to heat shock proteins in autoimmune arthritis." Database accession no. PREV199800228934 XP002136641 & BIOTHERAPY (DORDRECHT) 1998, vol. 10, no. 3, 1998, pages 213-221, ISSN: 0921-299X
- GEORGE J ET AL: "Atherosclerosis-related markers in systemic lupus erythematosus patients: The role of humoral immunity in enhanced atherogenesis." LUPUS 1999, vol. 8, no. 3, 1999, pages 220-226, XP000906739 ISSN: 0961-2033

## Description

The present invention relates to an immunological and oral tolerance inducing composition according to the preamble of claims 1, 2, 3, 4 and concerns also the use of an orally administered immunological and oral tolerance inducing composition according to the preamble of claims 14, 15 and 17.

Atherosclerosis and its clinical sequelae represent one of the most hazardous insults on Western society in terms of morbidity and mortality. Hence, it is not surprising that immense efforts have been put forward to investigate its etiopathogenesis aiming to provide means of slowing or halting its early indolent progression. Although no definite answers exist it is becoming apparent that not one individual mechanism is solely responsible for the evolvement of the atherosclerotic plaque as was formerly presumed with regard to dysregulation of the lipid balance.

Clinicians use the expression atherosclerosis to define a histopathological process which results in occlusion of arteries in different parts of the body. The occlusion is caused by the accumulation of different cells of various origins which are gradually being filled with large amounts of fat from the circulation. The fat which mainly consists of 'bad' cholesterol low density lipoprotein (LDL), is the principal material which deposits in the vessel walls. In the later stages of atherosclerosis, the lipid filled zones begin to accumulate calcium. The deposition of calcium renders the arteries stiffer and less flexible, thereby causing their 'stony' appearance, an appearance which has led to the well-known expression of "sclerosis of the arteries" (arteriosclerosis). When the involved arteries block the blood flow to the heart, a person is afflicted with a 'heart attack'; when the brain arteries occlude, the person experiences a stroke. When arteries to the limbs narrow, the result is severe pain, decreased physical mobility and possibly the need for an amputation.

It is clear that the progression of the above-mentioned diseases is non-detectable for many years and only manifests themselves at advanced stages. When these manifestations become detectable it is much more difficult to offer a remedy, due to the advanced stages of the disease.

As mentioned above, atherosclerosis is the result of the deposit of fat in the walls of blood vessels, thereby creating a layer of atherosclerotic plaque, said layer clogs the flow of blood to vital organs, thereby leading to cerebrovascular accidents, myocardial infarctions or peripheral blood vessel diseases. Known risk factors for rapid progression of the condition include high blood pressure, diabetes, overweight, smoking and the lack of physical exercise.

In recent studies it appears that infectious factors such as Cytomegalo virus (CMV), Epstein-Bar (EBV) and Chlamydia pneumonia may also be involved in the progression of atherosclerosis. The above has been demonstrated by linking atherosclerosis with gingivitis, in addition to the correlation between vessel obstruction and restenosis and the titers of autoantibodies to anti-CMV antigens, or finding infectious particles in the atherosclerotic plaque.

In the last decade a body of evidence has been accumulated to support the theory that atherosclerosis has a significant infectious-autoimmune component.

In autoimmune diseases the immune system attacks our body components (autoantigens), in addition to attacking external invaders. The autoimmune diseases are classified as autoantibody mediated or cell mediated diseases. Typical autoantibody mediated autoimmune diseases are myasthenia gravis and idiopathic thrombocytopenic purpura (ITP), while typical cell mediated diseases are Hashimoto's thyroiditis and Diabetes type I.

### Involvement of the immune network in atherosclerosis

The recognition that immune mediated processes prevail within atherosclerotic lesions stemmed from the consistent observation of lymphocytes and macrophages in the earliest stages, namely the fatty streaks. These lymphocytes which include a predominant population of CD4+ cells (the remainder being CD8+cells) were found to be more abundant over macrophages in early lesions, as compared with the more advanced lesions, in which this ratio tends to reverse. These findings posed questions as to whether they reflect a primary immune sensitization to a possible antigen or alternatively stand as a mere epiphenomenon of a previously induced local tissue damage. Regardless of the factors responsible for the recruitment of these inflammatory cells to the early plaque they seem to exhibit an activated state manifested by concomitant expression of MHC class II HLA-DR and interleukin (IL) receptor as well as leukocyte common antigen (CD45R0) and the very late antigen 1 (VLA-1) integrin.

The on-going inflammatory reaction in the early stages of the atherosclerotic lesion may either be the primary initiating event leading to the production of various cytokines by the local cells (i.e endothelial cells, macrophages, smooth muscle cells and inflammatory cells), or it may be that this reaction is a form of the body's defense immune system towards the hazardous process. The cytokines which have been shown to be upregulated by the resident cells include TNF-α, IL-1, IL-2, IL-6, IL-8, IFN-γ and monocyte chemoattractant peptide-1 (MCP-1). Platelet derived growth factor (PDGF) which is expressed by all cellular constituents within atherosclerotic plaques have also been shown to be overexpressed, thus possibly intensifying the preexisting inflammatory reaction by a co-stimulatory support in the form of a mitogenic and chemotactic factor. Very recently, Uyemura K, Demer LL, Castle SC et al. Cross regulatory roles of IL-12 and IL-10 in atherosclerosis. J Clin Invest 1996 97; 2130-2138 have elucidated type 1 T-cell cytokine pattern in human atherosclerotic lesions exemplified by a strong expression of IFN-γ but not IL-4 mRNA in comparison with normal arteries. Furthermore, IL-12 - a T-cell growth factor produced primarily by activated moncytes and a selective inducer of Th1 cytokine pattern, was found to be overexpressed within lesions as manifested by the abundance of its major heterodimer form p70 and p40 (its dominant inducible protein) mRNA.

Similar to the strong evidence for the dominance of the cellular immune system within the atherosclerotic plaque, there is also ample data supporting the involvement of the local humoral immune system. Thus, deposition of immunoglobulins and complement components have been shown in the plaques in addition to the enhanced expression of the C3b and C3Bi receptors in resident macrophages.

Valuable clues with regard to the contribution of immune mediated inflammation to the progression of atherosclerosis comes from animal models. Hence, it seems that immunocompromised mice (class I MHC deficient ) tend to develop accelerated atherosclerosis as compared with immune competent mice. Additionally, treatment of C57BL/6 mice (Emeson EE, Shen ML. Accelerated atherosclerosis in hyperlipidemic C57BL/6 mice treated with cyclosporine A. Am J Pathol 1993; 142: 1906-1915) and New-Zealand White rabbits (Roselaar SE, Schonfeld G, Daugherty A. Enhanced development of atherosclerosis in cholesterol fed rabbits by suppression of cell mediated immunity. J Clin Invest 1995; 96: 1389-1394) with cyclosporine A, which is- a potent suppressor of IL-2 transcription resulted in a significantly enhanced atherosclerosis under "normal" lipoprotein "burdeon". These latter studies may provide insight into the possible roles of the immune system as engaged in counteracting the self-perpetuating inflammatory process within the atherosclerotic plaque.

Atherosclerosis is not a classical autoimmune disease, although some of its manifestations such as the production of the plaque which obstruct the blood vessels may be related to immune system effects. In classical autoimmune disease, one can also define very clearly the autoantigen attacked by the immune system, one can define the part of the immune system which attacks the autoantigen (autoantibody or cells) which belong to the immune system and are named lymphocytes. Above all one can show that by passive transfer of these components of the immune system the disease can be induced in healthy animals, or in the case of humans the disease may be transferred from a sick pregnant mother to her offspring. Many of the above are not prevailing in atherosclerosis. In addition, the disease definitely has common risk factors such as hypertension, diabetes, lack of physical activity, smoking and others, the disease affects elderly people and has a different genetic preponderance than in classical autoimmune diseases.

The process of inducing oral tolerance has been known since the beginning of the century for reducing allergic reactions. The allergic patient was fed with a low dose of the known allergen and the body's immune tolerance was restored and no allergic reaction occurred.

### Oral tolerance in autoimmune diseases

In autoimmune diseases oral tolerance is a term denoted to describe blunting the immune response in an autoimmune disease. The tolarization produced by feeding an animal with the 'incriminated' antigen, can abrogate the development of the disease due to a 'paralyzing' effect on the immune response. The present inventors have recently shown that oral feeding with human β2GPI prior to immunization with this molecule, resulted in amelioration of the anti-phospholipid (APS)-equivalent syndrome.

Oral tolerance has been applied in the realm of autoimmune diseases, in which an immune reaction was carried out against the autoantigen and there existed a need for restoring the immune system's tolerance to the autoantigen. This was carried out by feeding a subject with low doses of the autoantigen. So far, several animal models have been reported in which oral tolerance has been restored, including preventing the development of experimental allergic encephalomyelitis (EAE), which is the equivalent of multiple sclerosis (MS), by feeding a subject with a protein from the nerve membrane called myelin basic protein (MBP), in addition to preventing adjuvant arthritis and collagen arthritis, by feeding a subject with collagen and HSP-65, respectively. A Boston based company called Autoimmune has carried out several human experiments for preventing diabetes, multiple sclerosis, rheumatoid arthritis and uveitis. The results of the human experiments have been less impressive than the non-human ones, however there has been a success with the prevention of arthritis.

### Antigenic Components

### A. Oxidized low density lipoprotein

LDL is a complex of large molecular weight proteins, including apolipoprotein B, neutral and polar lipids and lipophilic antioxidants (vitamin E and β carotene). The oxidation-modification of LDL with the resultant formation of neoepitopes within the native molecule (within the Apo B domain) leads to its recognition by the scavenger receptor on the macrophages. It is possible that all cellular components in the atherosclerotic plaque (i.e. endothelial cells, macrophages, smooth muscle cells, lymphocytes) are capable of enhancing lipid peroxidation with the production of varying degrees of LDL oxidation, yet the relative contribution of each, has not been determined. Regardless of the precise roles of these cells it appears that the balance between the oxidant and antioxidant forces at the level of the vessel walls determines the extent of exposure to Ox LDL with the subsequent deleterious effects. Ox LDL has active derivatives such as lysophosphatidylcholine (LPC) and, despite having a smaller molecular weight, still retains some of its biological activities.

Lysophosphatidylcholine (LPC) is expressed in human atherosclerotic plaques. It is an active biological substance that can induce the first steps of atherogenesis. Indeed it is even more potent than Ox LDL.

The overall *in-vivo* and *in-vitro* influence of Ox LDL and its byproducts on the development of the plaque are well beyond the scope of the present invention, however it is important to state the known relationship between Ox LDL and the immune system.

It is known that Ox LDL is chemotactic for T-cells and monocytes. Ox LDL and its byproducts are also known to induce the expression of factors such as monocyte chemotactic factor 1, secretion of colony stimulating factor and platelet activating properties, all of which are potent growth stimulants.

The active involvement of the cellular immune response in atherosclerosis has recently been substantiated by Stemme S, Faber B, Holm J. T lymphocytes from human atherosclerotic plaques recognize oxidized low density lipoprotein. Proc Natl Acad Sci USA 1995; 92: 3893-97, who isolated CD4+ within plaques clones responding to Ox LDL as stimuli. The clones corresponding to Ox LDL (4 out of 27) produced principally interferon-y rather than IL-4. It remains to be seen whether the above T-cell clones represent mere contact with the cellular immune system with the inciting strong immunogen (Ox LDL) or that this reaction provides means of combating the apparently indolent atherosclerotic process.

The data regarding the involvement of the humoral mechanisms and their meaning are much more controversial. Several reports have associated increased levels of antibodies to Ox LDL with the progression of atherosclerosis (expressed by the degree of carotid stenosis, severity of peripheral vascular disease etc.). However, these data were not reproduced by other scientists, perhaps due to the lack of standardization with regard to the assays used for determining antibodies to LDL. In any case there seems to be a consensus as to the presence of Ox LDL antibodies in the form of immune complexes within atherosclerotic plaque, although the true significance of this finding has not been established.

Antibodies to Ox LDL have been hypothesized as playing an active role in lipoprotein metabolism. Thus, it is known that immune complexes of Ox LDL and its corresponding antibodies are taken up more efficiently by macrophages in suspension as compared with Ox LDL. No conclusions can be drawn from this consistent finding on the pathogenesis of atherosclerosis since the question of whether the accelerated uptake of Ox LDL by the macrophages is beneficial or deleterious has not yet been resolved.

Important data as to the significance of the humoral immune system in atherogenesis comes from animal models. It has been found that hyperimmunization of LDL-receptor deficient rabbits with homologous oxidized LDL, resulted in the production of high levels of anti-Ox LDL antibodies and was associated with a significant reduction in the extent of atherosclerotic lesions as compared with a control group hyperimmunized with phopsphate-buffered saline (PBS). A decrease in plaque formation has also been accomplished by immunization of rabbits with cholesterol rich liposomes with the concomitant production of anti-cholesterol antibodies, yet this effect was accompanied by a 35% reduction in very low density lipoprotein cholesterol levels. The present inventors have shown that in apoE knockout mice, repeated immunizations with homologous Ox LDL results in production of anti-Ox LDL antibodies and in reduced atherosclerosis.

### B. Heat Shock protein (HSP) 60/65

An additional major antigenic component for the initiation and perpetuation of the inflammatory lesion culminating in enhanced atherosclerosis is the 60 Kd heat shock protein. This mitochondrial protein is a member of the HSP family which constitutes about 24 proteins displaying high degree of sequence homologies between different species. These proteins, as their name implies are upregulated in response to various stressful insults including exposure to free radicals, heat, mechanical shear stress, infections, cytokines etc. The teleological importance of the HSP stems from their protective roles against unfolding of cellular proteins precipitated by stressful stimuli. This role has led to their designation as molecular 'chaperones'. However, these apparently favorable effects of HSP's can be a double-edged sword, since their overexpression may, under certain conditions promote an autoimmune reaction with resultant tissue damage. The mechanisms responsible for the HSP immune mediated damage are not completely understood and it is presumed that cryptic neoepitopes are exposed to the immune system which no longer consider them as 'self following their upregulation. Alternatively, it was suggested that cross reaction exists between 'foreign' HSP and self HSP introduced following infections which act to trigger an immune attack against self structures.

HSP 60 is a distinct protein which constitutes a separate "class" within the above-mentioned HSP family, together with HSP 65, providing a sequestered environment for folding a subset of proteins in-vivo.

There exists a similarity between mammalian HSP60 and bacterial HSP65, which allows for cross recognition by immune effectors of the host.

Support for the involvement of HSP in autoimmunity is provided by studies documenting enhanced autoantibody as well as cellular response to HSP 60/65 in several autoimmune diseases.

The link between HSP 65 and atherosclerosis was initially raised following a pioneering work conducted by Georg Wick's group in the early 1990s (Xu Q, Dietrich H, Steiner HJ & al. Induction of arteriosclerosis in normocholesterolemic mice rabbits by immunization with heat shock protein 65. Arteriosclerosis Thrombosis 1992; 12: 789-799). They found that rabbits immunized with different antigens developed atherosclerosis, provided the preparation used for immunization contained complete Freund's adjuvant (CFA). Since the major constituent of CFA is heat killed mycobacterium tuberculosis the principal component of which is the HSP-65, they reasoned that the immune response towards this component led to the development of atherosclerosis. This hypothesis was confirmed when the animals were subsequently immunized merely with HSP-65 and exhibited pronounced atherosclerosis. It should be emphasized that no difference was noted between the groups with regard to the lipoprotein profile. Additional work conducted later by Wick's group revealed that T cells extracted from lesions of the rabbits induced to develop atherosclerosis were found to overexpress HSP-65 in comparison with peripheral blood from the respective animals thus attesting for a localized and restricted immune reaction in the vicinity of the stressed arterial vessel. The present inventors have reinforced Wick's finding, by showing that HSP-65 (or Mycobacterium tuberculosis) immunization of naive mice resulted in accelerated fatty streak formation.

These findings were further substantiated when involvement of humoral immune mechanisms in response to HSP-65 were observed in patients inflicted with atherosclerosis. Hence, a correlation has been established between high levels of anti-HSP65 antibodies and the extent of sonographically estimated carotid narrowing in a screen of healthy individuals.

These findings were recently reinforced by *in-vitro* assays designed to assess the influence of HSP-60/65 on cultured endothelial cells. Accordingly, it has been shown that incubation of endothelial cells with HSP65 induced their adhesiveness to monocytes and granulocytes which was concentration and time dependent. Furthermore, it has been demonstrated that this effect is mediated by overexpression of endothelial cells E-selectin (C62E), vascular cell adhesion molecule-1 (CD106) and intracellular adhesion molecule-1 (CD54).

### Phospholipids and β2GPI as autoantigen components in atherosclerosis.

The present inventors have found that the production of anticardiolipin in LDL-receptor knockout mice by immunization with human anticardiolipin antibodies (aCL) results in accelerated early atherosclerosis. This observation is consistent with circumstatial data that has accumulated with regard to the possible proatherogenic role of anticardiolipin antibodies in atherosclerosis.

β2GPI is a molecule that was proved as the target of aCL from patients with autoimmune diseases. Anti-β2GPI antibodies have been shown to result in experimental APS and to activate endothelial cells and platelets. The present inventors have shown that trasgenic LDL-RD deficient mice immunized with β2GPI develop respective antibodies and that early fatty streak formation is enhanced, accompanied by local accumulation of CD4+ cells. Thus, β2GPI may serve as an autoantigenic target of the autoimmune response in atherosclerosis.

In both animal and human disease models a correlation has been found between the titer autoantibodies to the autoantigens HSP 60-65 and Ox LDL and the level of atherosclerosis. The Ox LDL is produced in the body by the oxidation of the native LDL and is considered the noxious factor of atherosclerosis. In response to said toxin, anti-Ox LDL antibodies are produced. The β2GP-I is a natural protein in human blood which plays a role in the coagulation process and HSP65 is the result of various stress stimuli.

In experiments carried out with mice having a predisposition for the development of atherosclerosis (LDL-R knock-out mice), a thirty percent reduction was demonstrated in the atherosclerosis condition by feeding the mice with low doses of Ox LDL.

Furthermore, the fact that the immune system is involved in the induction of atherosclerosis, as well as the clearcut involvement of the immune system (lymphocytes and autoantibodies), points to the ability to manipulate the disease by inducing oral tolerance with autoantigens, e.g., Ox LDL, HSP60/65 and β₂GPI.

U.S. Patent, No. 5,348,945 discloses a method of combating mortality in a cell or tissue under stress. The method comprises contacting heat shock protein 70 (HSP70) to the cell or tissue in an amount effective to enhance the survival of that cell or tissue. The method may be employed in the combating of atherosclerosis, restenosis after angioplasty and nerve damage in human or animal subjects in need of such treatment. A pharmaceutical composition comprising a therapeutically effective amount of HSP70 in a pharmaceutically acceptable formulation is also disclosed.

Although HSP70 and HSP60 belong to a family of about 24 highly conserved heat shock proteins, they represent two entirely distinct characteristics. Their mechanism, for example, do not appear to act in concert in governing the protection from stressful stimuli.

HSP70 was initially patented because of its pronounced induction during heat exposure and other stressful insults such as ischemic preconditioning. Indeed the overexpression of HSP70 in transgenic animals is associated with protection from stressful hazards.

Therefore, U.S. Patent No. 5,348,945 does not teach or suggest the subject matter of the present invention.

The immunological and oral tolerance inducing composition of the present invention is defined in the characterizing part of claims 1, 2, 3 and 4 and the use of an orally administered immunological and oral tolerance inducing composition is defined in the characterizing part of claims 14, 15, 16 and 17.

Thus, according to the present invention there is now provided an immunological and oral tolerance-inducing composition for prevention and/or theatment of atherosclerosis by the oral administration thereof, comprising an active component selected from the group consisting of modified low density lipoprofein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

In an preferred embodiment of the present invention there is provided an immunological and oral tolerance-inducing composition for prevention and/or treatment of a heart attack by the oral administration thereof, comprising an active component selected from the group consisting of modified low density lipoprotein, oxidized low density lipoprotein (Ox LDL), heat shock protein 60/65. (HSP 60/65), beta₂-glycoprotein-1 (β₂GP-1), functional derivatives thereof and mixtures thereof, in combination with a pharmaceutically acceptable carrier for oral administration.

In another preferred embodiment of the present invention there is provided an immunological and oral tolerance-inducing composition for prevention and/or treatment of angioplasty-restenosis by the oral administration thereof, comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

In a further preferred embodiment of the present invention there is provided an immunological and oral tolerance-inducing composition for prevention and/or treatment of stroke by the oral administration thereof, comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

In even further preferred embodiments of the present invention there is provided an immunological and oral tolerance-inducing composition wherein said active component is a modified low-density lipoprotein, or wherein said active component is oxidized low-density lipoprotein (Ox LDL), or wherein said active component is an active derivative of oxidized low-density lipoprotein (Ox LDL), or wherein said active component is heat shock protein 60/65 (HSP 60/65), or wherein said active component is beta₂-glycoprotein-1 (β₂GP-1).

The present invention provides an immunological and oral tolerance-inducing composition, wherein said active derivative is lysophosphatidyl choline (LPC).

The present invention also provides an immunological and oral tolerance-inducing composition, wherein said LDL is malondialdehyde LDL (MDA-LDL).

In another aspect of the present invention there is provided the use, for the manufacture of a medicament for prevention and/or treatment of atherosclerosis in a subject, of an immunological oral tolerance-inducing composition comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

In a preferred embodiment there is provided the use, for the manufacture of a medicament for prevention and/or treatment of a heart attack in a subject, of an immunological oral tolerance-induang composition comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

In a further preferred embodiment there is provided the use, for the manufacture of a medicament for prevention and/or treatment of angioplasty-restenosis following angioplasty in a subject, of an immunological oral tolerance-inducing composition comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

In an even further preferred embodiment there is provided the use, for the manufacture of a medicament for prevention and/or treatment of stroke in a subject, of an immunological oral tolerance-inducing composition comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

In further embodiments of the present invention there is provided the use, for the manufacture of a medicament for prevention and/or treatment of atherosclerosis in a subject, wherein said active component is a modified low-density lipoprotein, or wherein said active component is oxidized low-density lipoprotein (Ox LDL), or wherein said active component is an active derivative of oxidized low-density lipoprotein (Ox LDL), or wherein said active component is heat shock protein 60/65 (HSP 60/65), or wherein said active component is beta₂-glycoprotein-1 (β₂GP-1).

The present invention further provides the use, for the manufacture of a medicament for prevention and/or treatment of atherosclerosis in a subject, wherein said active derivative is lysophosphatidyl choline (LPC).

The present invention further provides the use, for the manufacture of a medicament for prevention and/or treatment of atherosclerosis in a subject, wherein said LDL is malondialdehyde LDL (MDA-LDL).

The term "functional derivative" as used herein is intended to include labelled proteins, conjugated proteins, fused chimeric proteins and purified receptors in soluble form, as well as fragments, deletions, and conservative substitutions of said proteins.

The existence of an immune response against Ox LDL in atherosclerosis and the correlation between the reaction to Ox LDL and the severity of the disease, in combination with evidence that an active vaccine of Ox LDL in mice and rabbits can prevent the development of atherosclerosis has led the present inventors to conclude that the induction of immune tolerance by feeding Ox LDL to a human subject can result in the reduced rate of atherosclerosis progression. It should be mentioned that the mechanisms of inducing immune tolerance by mouth feeding are possibly mediated via a stimulation and production of cytokine TGFβ and the development of non-specific suppresser T-cells.

The oral tolerization of the present invention may extend to yield a bystandard suppression effect: namely - blocking other (non-antigen specific) autoimmune (anti-self) responses occurring in the vicinity of the atherosclerotic plaque and contributing to its progression.

It should be noted that the aim of the present invention is to induce tolerization or paralyze the immune response towards the HSP65, rather than to achieve mere elevation in the serum to assist protein unfolding.

Therefore, in one aspect the present invention combines oral tolerance, Ox LDL and atherosclerosis, which is a disease caused in part by immune factors. Ox LDL has been reported to induce an immune reaction in mice and rabbits (in contrast to inducing immune tolerance) of Ox LDL antigens and an improvement in the atherosclerosis condition. In these animal models Ox LDL has not been reported to have been experimented with mouth feeding and has never been suggested for oral tolerance.

The pharmaceutical compositions for oral administration according to the present invention are prepared by methods known per se and the administration thereof is by known methods of oral administration.

An amount effective to treat the disorder hereinbefore described depends on the usual factors such as the nature and severity thereof and the weight of the mammal.

For oral administration, it is preferred that the active ingredient be administered in the form of a unit-dose composition.

Such compositions are prepared by admixture and are suitably adapted for oral administration in the form of tablets, capsules, oral liquid preparations, powders, granules, etc.

Tablets and capsules for oral administration are usually presented in a unit dose, and contain conventional excipients such as binding agents, fillers, diluents, tabletting agents, lubricants, disintegrants, colorants, flavorings, and wetting agent. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycolate. Suitable lubricants include, for example, magnesium stearate. Suitale pharmaceutically acceptable wetting agents include sodium lauryl sulphate.

These solid oral compositions may be prepared by conventional methods of blending, filling or tabletting. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or with another suitable vehicle before use. Such liquid preparations may contain conventional additives such as suspending agents, for example, sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or proply p-hydroxybenzoate or sorbic acid, and if desired conventional flavoring or coloring agents.

Oral formulations also include conventional sustained release formulations, such as tablets or granules having an enteric coating.

### Description of Preferred Embodiments

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### MATERIALS AND METHODS

### Animals.

Twelve weeks old LDL-RD mice (hybrids of between the C57BL/6J and 129Sv strains) are created by homologous recombination. The mice are purchased from The Jackson Laboratory (Bar-Harbor ME). The LDL-RD mice are used since they have high cholesterol levels on chow diet (which are similar to human values) and develop significant atherosclerosis only when fed a high fat diet. The LDL-RD mice are either fed with a normal chow-diet containing 4.5% fat by weight (0.02% cholesterol) or an atherogenic diet (Harlan, Teklad Premier Laboratory Diets, Madison, WI) containing: 1.25% cholesterol, 7.5% casein and 0.5%(wt/) sodium-cholate. The mice are maintained on 12 hour-dark/12 hour light cycles and are allowed to access food and water *ad libitum.*

### LDL isolation, oxidation and characterization

Blood for lipoprotein isolation is collected in EDTA (1mg/ml) after 12 hours of fasting. LDL (density=1.019-1.063 g/l) is isolated from the plasma after density adjustment with Kbr⁻, by preparative ultracentrifugation at 50,000 rpm/min for 22 hours, using type 50 rotor. The LDL preparations are washed by ultracentrifugation, dialyzed against a pH 7.4, 0.15 mol/L EDTA, passed through Acrodisc filter (0.22 um pore size) to remove aggregates, and stored under nitrogen in the dark.

LDL oxidation is performed by incubation of pre-dialyzed LDL (1 mg of protein/ml in EDTA-free PBS) with copper sulfate (10µM) for 24 h at 37° C. Lipoprotein oxidation is confirmed by analysis of thiobarbituric acid-reactive substances (TBARS) which measures malondialdehyde (MDA) equivalents by the lipid peroxidation test and also by analysis of the conjugated diene content of the lipoprotein.

### Determination of anti-Ox LDL antibody titers.

Preparation of native and copper-oxidized LDL is preformed. Ninety six well polystyrene plates (Nunc Maxisorp, Denmark) are coated with either Ox LDL, native LDL (at concentration of 5 ug/ml in PBS) or PBS overnight at 4°C. After washing four times with PBS containing 0.05% Tween and 0.001% aprotinin (Sigma, USA) the plates are blocked with 2% bovine serum albumin (BSA) for 2 hours at room temperature. Serum fractions are diluted to 1:50 in PBS 0.05% Tween 0.2% BSA. After an additional overnight incubation the plates are washed and alkaline phosphatase-conjugated goat anti-mouse IgG (Jackson ImmunoResearch laboratory Inc., USA) are added and diluted to 1:10,000 in PBS 0.05% Tween-0.2% BSA for 1 hour at room temperature. After extensive washing, 1 mg/ml *p*-nitrophenyl-phosphate (Sigma) in 50 mM 'carbonate buffer containing 1mM MgCl₂ pH 9.8 is added as a substrate. The reaction is stopped after 30 min by adding 1 M of NaOH. The color is read at a 405 nm wavelength in a Titertek ELISA reader (S.L.T Laboratory Instruments, Vienna, Austria) and results expressed as O.D. at 405 nm. The titer of anti-Ox LDL antibodies is calculated by subtracting the value obtained from binding to native LDL from the binding to Ox LDL.
Inhibition assays are performed to confirm the specificity of anti-Ox LDL antibodies and to check for a possible cross-reactivity with HSP-an important immunogen in atherosclerosis. The concentration of HSP-65-immunized mouse serum giving half of the maximal binding to Ox LDL are determined and different inhibitors (i.e. HSP-65, Ox LDL, bovine serum albumin) are applied in increasing concentrations.

### Detection of Anti-HSP-65 Antibodies.

Recombinant HSP-65 (1µg/ml) in phosphate buffered saline (PBS, pH 7.2) are coated onto flat bottom 96-well ELISA plates (Nunc Maxisorp, Denmark) by overnight incubation at 4°C. After washings with 0.02% PBS Tween and blocking with 1% BSA in PBS, sera are added in different dilutions (1:50, 1:100, 1:200, in PBS) and incubated for 1hr at room temperature. Peroxidase conjugated rabbit anti-mouse IgG (Dako Ltd, High Wycombe, UK) are added and incubated for 1 hr at room temperature followed by 4 washings with PBS/Tween. Finally, 100µl of citrate phosphate buffer (0.1 M, pH 4.2) containing 0.53 mg/ml of 2,2-azino-bis 3-ethylbenzthiazoline-6-sulfonic acid (ABTS; Sigma) are added, and absorbance is measured after 30 min at 490 nm in a Titertek ELISA reader.

### Detection of anti-phospholipid antibodies.

A modified ELISA to determine reactivity of the mouse and human antibodies to PL is perfrormed. Microtiter plates (Nunc, Maxisorp, Denmark) are coated either with an anionic PL [cardiolipin (CL), phosphatidylserine (PS), phosphatidylinositol (PI)] and phosphatidylcholine (PC), all from Sigma Chemicals Co. St. Louis, Mo, at a concentration of 50 ug/ml and dissolved in ethanol, except for PC (choloroform-methanol, 1: 3). Plates are dried under vacuum and blocked with TBS containing 0.5% gelatin. The plates are then washed three times with TBS and different concentrations of mouse preparations are added to the wells treated with human β2GPI (5ug/ml for 30 min) or 0.1% gelatin/TBS alone. The binding of the antibodies is detected with goat anti-mouse alkaline phosphatase conjugate and the addition of substrate (*p*-nitro-phenylphosphate). The results are expressed as absorbance at 405 nm (OD₄₀₅).

### Detection of anti-b2GPI antibodies.

One ug/ml β2GPI or a different DM is incubated overnight in 50 mM bicarbonate buffer, pH 9.6 at 4°C in 96-well polystyrene plates (Nunc). After 3 washings with TBS, blocking is performed with 0.5% gelatin/TBS for 2 hrs (as for the anti-PL ELISA). The plates are then washed three times and murine sera (diluted in 1: 100 in gelatin/TBS) is added and incubated for 2 hrs at room temperature. After 3 washings, alkaline phosphatase conjugated goat anti-human IgG respectively diluted in 0.1% gelatin/TBS (1:10,000) is added for 2 hrs. After 3 additional washings, the substrate *p*-nitrophenylphosphate in a sodium carbonate buffer pH 9.8 is added and absorbance is read at 405 nm.

### Proliferation Assays of Spleen Lymphocytes.

Spleens are removed from mice upon sacrifice, and lymphocytes are isolated. 1x10⁶ cells per ml are incubated in triplicates for 72 h in 0.2 ml of culture medium in microtiter wells in the presence of different concentrations of HSP-65, Ox LDL, β2GPI or ovalbumin. Proliferation is measured by the incorporation of [³H] thymidine into DNA during the final 12 hours of incubation. The results are computed as stimulation index (S.I.): the ratio of the mean cpm of the antigen to the mean background cpm obtained in the absence of the antigen.

### Cytokine level determination

Spleen cells are removed following sacrifice and splenocytes are incubated for 3 days with Ox LDL, HSP-65 or ovalbumin following which the supernatant is collected . Cytokine profile (IL-4, IL-10, IFN-γ, and TGF-β) is determined at the cultured supernatant of the splenocytes.

### Examples

### Example 1

The mice used in this experiment are called LDL-receptor deficient (LDL-RD) mice. These animals have a genetic mutation which causes a defect in the receptor for LDL in all cells of the body. This receptor is responsible for evacuating and removing from the circulation, the 'bad' cholesterol (LDL), and when it is deficient the mice become hyperlipidemic and develop atherosclerosis when they are fed a high fat diet for 3-5 weeks.

Three groups of mice were used (15 LDL-RD mice in each group). Feeding was performed by a special device (canula) designed to be introduced to the esophagus and thus assuring the fed substances reaches mostly to the stomach.
**Group 1:** The mice were fed 5 doses of 1mg of human Ox LDL dissolved in PBS, every other day. At the end of the last dose, the mice were put on a high fat diet and sacrificed either after 3 or after 5 weeks of diet.
**Group 2**: The mice were fed 5 does of 1 mg of a control protein (ovalbumin) in PBS and then fed with the special diet for 3 or 5 weeks.
**Group 3**: The mice were not fed prior to initiation of the diet.

The mice were evaluated, upon sacrifice for the occurrence of atherosclerotic lesions, serum cholesterol values and levels of antibodies to Ox LDL.
**Results**: All mice were of similar weight prior to, and at the termination of the study. The mice fed OX LDL were found to develop less atherosclerosis (30% less).

In the present studies the beneficial effect of oral tolerance with Ox LDL on the induction of atherosclerosis in mice has been shown. There are many studies in which the results point to the effectiveness of oral tolerance in various autoimmune diseases, such as collagen in adjuvant arthritis (the analogue of rheumatoid arthritis), in diabetes mellitus, in uveitis, in EAE (analogue to multiple sclerosis). In the above-mentioned studies human trial was done. Recently a remarkable effect was noted when patients with rheumatoid arthritis were given per oral collagen II (oral tolerance). The comparable effect between mice studies and humans in various autoimmune diseases point to the extrapolated success in humans. It should be stressed that this treatment has no side effects.

### Example 2

### Suppression of high fat diet induced atherosclerosis in LDL-receptor deficient mice by feeding with human Ox LDL

LDL-RD mice (n=15) are fed 5 doses (every other day) of human Ox LDL in three different concentrations (1mg/dose, 100ug/dose and 1mg/dose). Additional LDL-RD mice (n=15 per groups) are fed a control antigen (ovalbumin at similar doses).

One day following the last feeding, all mice are challenged with a high fat diet ('paigen') for 5 weeks.

At the end of the experiment, sera from all mice is evaluated for the presence of anti-Ox LDL, anti-HSP65 and antiphospholipid antibodies and a lipid profile is carried out (total cholesterol, LDL, VLDL, HDL and triglycerides).

Hearts from all mice are removed at the end of the study and frozen in -70°C until use (see Materials and methods) Immunohistochemical studies on the cryostat sections are performed using monoclonal antibodies to CD3, CD4, CD8, macrophages, smooth muscle cells and lymphocyte activation markers.
A similar protocol of feeding is carried out using LPC an active derivative of Ox LDL.

### Example 3

### Suppression of Mycobaceterium tuberculosis (MT) induced atherosclerosis by feeding with HSP-65.

The present inventors have observed that LDL-RD mice immunized once, with MT develop enhanced early atherosclerosis .
LDL-RD mice (n=15) are fed 5 doses (every other day) of recombinant HSP65 in three different concentrations (100mg/dose, 10ug/dose and 1ug/dose). Additional LDL-RD mice (n=15 per groups) are fed a control antigen (ovalbumin at similar doses).

One day following the last feeding, all mice are challenged by an immunization with heat killed suspension of MT (10 mg/ml; 100ug/mouse) emulsified in incomplete Freund's adjuvant. The mice are sacrificed 12 weeks following the immunization with MT.

At the end of the experiment, sera from all mice is evaluated for the presence of anti-HSP65 antibodies, and a lipid profile is carried out (total cholesterol, LDL, VLDL, HDL and triglycerides).

Proliferative responses from draining lymph node cells are evaluated to HSP65 from HSP65 tolerized mice and from control fed mice.

Cytokine levels (IL-4, IFN-γ, IL-10 and TGF-β) are evaluated in the supernatant collected from the medium in which lymphocytes are induced in-vitro with HSP65.

Hearts from all mice are removed and frozen in -70°C. To determine the extent of atherosclerosis cryostat sections are performed (5um per section). The relevant sections (from the aortic sinus area) are stained with the Oil-red O and the extent of atherosclerosis is determined by counting the area counted by a grid.

Immunohistochemical studies on the cryostat sections are performed using monoclonal antibodies to CD3, CD4, CD8, macrophages, smooth muscle cells and lymphocyte activation markers.

### Example 4

### Suppression of atherosclerosis by feeding with human β2GPI

The procedure is similar to the one employed for Ox LDL and HSP-65 and is based on the observation that immunization with β2GPI induces early atherogenesis.

### Assessment of the extent of atherosclerosis.

Quantification of atherosclerotic fatty streak lesions are carried out by calculating the lesion size in the aortic sinus (with a few modifications). Briefly, the heart and upper section of the aorta are removed from the animals and the peripheral fat cleaned carefully. The upper section is embedded in OCT medium and frozen. Every other section (10µm thick) throughout the aortic sinus (400µm) is taken for analysis. The distal portion of the aortic sinus is recognized by the three valve cusps which are the junctions of the aorta to the heart. Sections are evaluated for fatty streak lesions after staining with oil-red O. Lesion areas per sections are counted using a grid by an observer unfamiliar with the tested specimen.

The extent of atherosclerosis is evaluated at the level of the aortic sinus. Processing and staining of the tissue is carried out. Lesion area is quantified by the modified method of Rubin EM, Kraus RM, Spangler EA & al. (Inhibition of early atherogenesis in transegenic mice by human apolipoprotein Al. Nature 1991; 353: 265-267).

### Immunohistochemistry of atherosclerotic lesions.

*mAbs:* Rat monoclonal antibodies *H129.19* (*L3T4*)-anti-mouse CD4+ and *S3-6.7 (Ly-2)*-anti-mouse CD8a are obtained from PharMingen (San Diego, CA, USA) ; *MCA 497 (F4*/*80)*-anti-mouse macrophages are obtained from Serotec (Oxford, UK).

Immunohistochemical staining for CD4, CD8 and macrophages are carried out on aortic sinus 5 µm thick frozen sections. The sections are fixed for 4 min. in methanol at -20°C followed by 10 min. incubation with ethanol at -20°C. The sections are then blocked with non-immune goat serum for 15 min. at room temperature followed by incubation with CAS blocking reagent for 30 min. at room temperature. Subsequently, the rat-monoclonal anti-mouse CD4/CD8 is added for 1 hr aroom. After washings, affinity purified biotinylated rabbit anti-rat IgG antibodies are added for 30 min at RT. After washings, the slides are incubated with 0.3% H₂O₂, followed by additional rinses and incubation with streptavidin-peroxidase conjugate for 30 min at RT. After washings, the slides are developed with 3 amino-9-ethylcarbonasole (AEC) substrate (Dako) for 15 min. Sections are counterstained with hematoxylin. Spleen sections are used as a positive control. Staining in the absence of 1st or 2nd antibody are used as a negative control.

### Adoptive transfer experiments

T-cells (either CD4 or CD8 cells) will be isolated from the tolerized and non tolerized mice in each of the studies and transferred to LDL-RD littermates which will be challenged with an atherogenic diet for 6 weeks. At the end of the experiments the hearts will be taken for evaluation of atherosclerosis and immunohistochemistry.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. An immunological and oral tolerance-inducing composition for prevention and/or treatment of atherosclerosis by the oral administration thereof, comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

2. An immunological and oral tolerance-inducing composition for prevention and/or treatment of a heart attack by the oral administration thereof, comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

3. An immunological and oral tolerance-inducing composition for prevention and/or treatment of angioplasty-restenosis by the oral administration thereof, comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivates thereof and mixtures thereof, in combination with a pharmaceutically acceptable carrier for oral administration.

4. An immunological and oral tolerance-inducing composition for prevention and/or treatment of stroke by the oral administration thereof, comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration.

5. An immunological and oral tolerance-inducing composition according to claim 1, wherein said active component is a modified low-density lipoprotein.

6. An immunological and oral tolerance-inducing composition according to claim 1, wherein said active component is oxidized low-density lipoprotein (Ox LDL).

7. An immunological and oral tolerance-inducing composition according to claim 1, wherein said active component is an active derivative of oxidized low-density lipoprotein (OX LDL).

8. An immunological and oral tolerance-inducing composition according to claim 1, wherein said active component is heat shock protein 60/65 (HSP 60/65).

9. An immunological and oral tolerance-inducing composition according to claim 1, wherein said active component is an active derivative of HSP 60/65.

10. An immunological and oral tolerance-inducing composition according to claim 1, wherein said active component is beta₂-glycoprotein-1 (β₂GP-1).

11. An immunological and oral tolerance-inducing composition according to claim 1, wherein said active component is an active derivative of β₂GP-1.

12. An immunological and oral tolerance-inducing composition according to claim 1, wherein said active component is an active derivative of LDL which active derivative is lysophosphatidyl choline (LPC).

13. An immunological and oral tolerance-inducing composition according to claim 1, wherein said LDL is malondialdehyde LDL (MDA-LDL).

14. Use of an orally administrable immunological and oral tolerance-inducing composition comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration for the manufacture of a medicament for treating and/or preventing atherosclerosis in a subject.

15. Use of an orally administrable immunological and oral tolerance-inducing composition comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration for the manufacture of a medicament for treating and/or preventing heart attack in a subject.

16. Use of an orally administrable immunological and oral tolerance-inducing composition comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration for the manufacture of a medicament for treating and/or preventing angioplasty-restenosis in a subject.

17. Use of an orally administrable immunological and oral tolerance-inducing composition comprising an active component selected from the group consisting of modified low density lipoprotein, oxidized low density lipoprotein (Ox LDL), heat shock protein 60/65 (HSP 60/65), beta₂-glycoprotein-1 (β₂GP-1), functional derivatives thereof and mixtures thereof, in combination with a pharmaceutically acceptable carrier for oral administration for the manufacture of a medicament for treating and/or preventing stroke in a subject.

18. The use according to any of claims 14-17 wherein said active component is a modified low-density lipoprotein.

19. The use according to any of claims 14-17 wherein said active component is oxidized low-density lipoprotein (Ox LDL).

20. The use according to any of claims 14-17 wherein said active component is an active derivative of oxidized low-density lipoprotein (Ox LDL).

21. The use according to any of claims 14-17 wherein said active component is heat shock protein 60/65 (HSP 60/65).

22. The use according to any of claims 14-17 wherein said active component is an active derivative of heat shock protein 60/65 (HSP 60/65).

23. The use according to any of claims 14-17 wherein said active component is beta₂-glycoprotein-1 (β₂GP-1).

24. The use according to any of claims 14-17 wherein said active component is an active derivative of beta₂-glycoprotein-1 (β₂GP-1).

25. The use according to any of claims 14-17 wherein said active component is an active derivative of LDL which active derivative is lysophosphatidyl choline (LPC).

26. The use according to any of claims 14-17 wherein said LDL is malondialdehyde LDL (MDA-LDL).

## Revendications

1. Composition induisant une tolérance immunitaire et orale pour la prévention et/ou le traitement de l'athérosclérose par l'administration orale de ladite composition, comprenant un composant actif choisi parmi le groupe constitué par les lipoprotéines de basse densité modifiées, les lipoprotéines de base densité oxydées (Ox LDL), la protéine de choc thermique 60/65 (HSP 60/65), la bêta₂-glycoprotéine-1 (β₂GP-1), leurs dérivés fonctionnels ainsi que leurs mélanges, en combinaison avec un support pharmaceutiquement acceptable pour une administration par voie orale.

2. Composition induisant une tolérance immunitaire et orale pour la prévention et/ou le traitement d'une crise cardiaque par l'administration orale de ladite composition, comprenant un composant actif choisi parmi le groupe constitué par les lipoprotéines de basse densité modifiées, les lipoprotéines de base densité oxydées (Ox LDL), la protéine de choc thermique 60/65 (HSP 60/65), la bêta₂-glycoprotéine-1 (β₂GP-1), leurs dérivés fonctionnels ainsi que leurs mélanges, en combinaison avec un support pharmaceutiquement acceptable pour une administration par voie orale.

3. Composition induisant une tolérance immunitaire et orale pour la prévention et/ou le traitement d'une resténose d'angioplastie par l'administration orale de ladite composition, comprenant un composant actif choisi parmi le groupe constitué par les lipoprotéines de basse densité modifiées, les lipoprotéines de base densité oxydées (Ox LDL), la protéine de choc thermique 60/65 (HSP 60/65), la bêta₂-glycoprotéine-1 (β₂GP-1), leurs dérivés fonctionnels ainsi que leurs mélanges, en combinaison avec un support pharmaceutiquement acceptable pour une administration par voie orale.

4. Composition induisant une tolérance immunitaire et orale pour la prévention et/ou le traitement de l'ictus par l'administration orale de ladite composition, comprenant un composant actif choisi parmi le groupe constitué par les lipoprotéines de basse densité modifiées, les lipoprotéines de base densité oxydées (Ox LDL), la protéine de choc thermique 60/65 (HSP 60/65), la bêta₂-glycoprotéine-1 (β₂GP-1), leurs dérivés fonctionnels ainsi que leurs mélanges, en combinaison avec un support pharmaceutiquement acceptable pour une administration par voie orale.

5. Composition induisant une tolérance immunitaire et orale selon la revendication 1, dans laquelle ledit composant actif est une lipoprotéine de basse densité modifiée.

6. Composition induisant une tolérance immunitaire et orale selon la revendication 1, dans laquelle ledit composant actif est une lipoprotéine de base densité oxydée (Ox LDL).

7. Composition induisant une tolérance immunitaire et orale selon la revendication 1, dans laquelle ledit composant actif est un dérivé actif d'une lipoprotéine de base densité oxydée (Ox LDL).

8. Composition induisant une tolérance immunitaire et orale selon la revendication 1, dans laquelle ledit composant actif est la protéine de choc thermique 60/65 (HSP 60/65).

9. Composition induisant une tolérance immunitaire et orale selon la revendication 1, dans laquelle ledit composant actif est un dérivé actif de HSP 60/65.

10. Composition induisant une tolérance immunitaire et orale selon la revendication 1, dans laquelle ledit composant actif est la bêta₂-glycoprotéine-1 (β₂GP-1).

11. Composition induisant une tolérance immunitaire et orale selon la revendication 1, dans laquelle ledit composant actif est un dérivé actif de β₂GP-1.

12. Composition induisant une tolérance immunitaire et orale selon la revendication 1, dans laquelle ledit composant actif est un dérivé actif du LDL, ledit dérivé actif étant la lysophosphatidylcholine (LPC).

13. Composition induisant une tolérance immunitaire et orale selon la revendication 1, dans laquelle ledit composant actif est un dérivé actif du LDL, ledit dérivé actif étant le LDL modifié par le dialdéhyde malonique (MDA-LDL).

14. Utilisation d'une composition induisant une tolérance immunitaire et orale pour la prévention et/ou le traitement de l'athérosclérose par l'administration orale de ladite composition, comprenant un composant actif choisi parmi le groupe constitué par les lipoprotéines de basse densité modifiées, les lipoprotéines de base densité oxydées (Ox LDL), la protéine de choc thermique 60/65 (HSP 60/65), la bêta₂-glycoprotéine-1 (β₂GP-1), leurs dérivés fonctionnels ainsi que leurs mélanges, en combinaison avec un support pharmaceutiquement acceptable pour une administration par voie orale pour la préparation d'un médicament destiné au traitement et/ou à la prévention de l'athérosclérose chez un sujet.

15. Utilisation d'une composition induisant une tolérance immunitaire et orale pour la prévention et/ou le traitement de l'athérosclérose par l'administration orale de ladite composition, comprenant un composant actif choisi parmi le groupe constitué par les lipoprotéines de basse densité modifiées, les lipoprotéines de base densité oxydées (Ox LDL), la protéine de choc thermique 60/65 (HSP 60/65), la bêta₂-glycoprotéine-1 (β₂GP-1), leurs dérivés fonctionnels ainsi que leurs mélanges, en combinaison avec un support pharmaceutiquement acceptable pour une administration par voie orale pour la préparation d'un médicament destiné au traitement et/ou à la prévention de la crise cardiaque chez un sujet.

16. Utilisation d'une composition induisant une tolérance immunitaire et orale pour la prévention et/ou le traitement de l'athérosclérose par l'administration orale de ladite composition, comprenant un composant actif choisi parmi le groupe constitué par les lipoprotéines de basse densité modifiées, les lipoprotéines de base densité oxydées (Ox LDL), la protéine de choc thermique 60/65 (HSP 60/65), la bêta₂-glycoprotéine-1 (β₂GP-1), leurs dérivés fonctionnels ainsi que leurs mélanges, en combinaison avec un support pharmaceutiquement acceptable pour une administration par voie orale pour la préparation d'un médicament destiné au traitement et/ou à la prévention de la resténose d'angioplastie chez un sujet.

17. Utilisation d'une composition induisant une tolérance immunitaire et orale pour la prévention et/ou le traitement de l'athérosclérose par l'administration orale de ladite composition, comprenant un composant actif choisi parmi le groupe constitué par les lipoprotéines de basse densité modifiées, les lipoprotéines de base densité oxydées (Ox LDL), la protéine de choc thermique 60/65 (HSP 60/65), la bêta₂-glycoprotéine-1 (β₂GP-1), leurs dérivés fonctionnels ainsi que leurs mélanges, en combinaison avec un support pharmaceutiquement acceptable pour une administration par voie orale pour la préparation d'un médicament destiné au traitement et/ou à la prévention de l'ictus chez un sujet.

18. Utilisation selon l'une quelconque des revendications 14 à 17, dans laquelle ledit composant actif est une lipoprotéine de basse densité modifiée.

19. Utilisation selon l'une quelconque des revendications 14 à 17, dans laquelle ledit composant actif est une lipoprotéine de base densité oxydée (Ox LDL).

20. Utilisation selon l'une quelconque des revendications 14 à 17, dans laquelle ledit composant actif est un dérivé actif d'une lipoprotéine de base densité oxydée (Ox LDL).

21. Utilisation selon l'une quelconque des revendications 14 à 17, dans laquelle ledit composant actif est la protéine de choc thermique 60/65 (HSP 60/65).

22. Utilisation selon l'une quelconque des revendications 14 à 17, dans laquelle ledit composant actif est un dérivé actif de la protéine de choc thermique 60/65 (HSP 60/65).

23. Utilisation selon l'une quelconque des revendications 14 à 17, dans laquelle ledit composant actif est la bêta₂-glycoprotéine-1 (β₂GP-1).

24. Utilisation selon l'une quelconque des revendications 14 à 17, dans laquelle ledit composant actif est un dérivé actif de la bêta₂-glycoprotéine-1 (β₂GP-1).

25. Utilisation selon l'une quelconque des revendications 14 à 17, dans laquelle ledit composant actif est un dérivé actif du LDL, ledit dérivé actif étant la lysophosphatidylcholine (LPC).

26. Utilisation selon l'une quelconque des revendications 14 à 17, dans laquelle ledit LDL est le LDL modifié par le dialdéhyde malonique (MDA-LDL).

## Patentansprüche

1. Eine immunologische und orale toleranzinduzierende Zusammensetzung zur Verhütung und/oder Behandlung von Arteriosklerose durch deren orale Verabreichung, umfassend eine aktive Komponente, gewählt aus der Gruppe bestehend aus modifiziertem niedrigdichtem Lipoprotein, oxidiertem niedrigdichtem Lipoprotein (Ox-LDL), Hitzeschockprotein 60/65 (HSP 60/65), Beta₂-Glucoproteid-1 (β₂GP-1), funktionellen Derivaten davon und Mischungen davon, in Kombination mit einem pharmazeutisch akzeptablen Träger zur oralen Verabreichung.

2. Eine immunologische und orale toleranzinduzierende Zusammensetzung zur Verhütung und/oder Behandlung eines Herzanfalls durch deren orale Verabreichung, umfassend eine aktive Komponente, gewählt aus der Gruppe bestehend aus modifiziertem niedrigdichtem Lipoprotein, oxidiertem niedrigdichtem Lipoprotein (Ox-LDL), Hitzeschockprotein 60/65 (HSP 60/65), Beta₂-Glucoproteid-1 (β₂GP-1), funktionellen Derivaten davon und Mischungen davon, in Kombination mit einem pharmazeutisch akzeptablen Träger zur oralen Verabreichung.

3. Eine immunologische und orale toleranzinduzierende Zusammensetzung zur Verhütung und/oder Behandlung von Angioplastierestenose durch deren orale Verabreichung, umfassend eine aktive Komponente, gewählt aus der Gruppe bestehend aus modifiziertem niedrigdichtem Lipoprotein, oxidiertem niedrigdichtem Lipoprotein (Ox-LDL), Hitzeschockprotein 60/65 (HSP 60/65), Beta₂-Glucoproteid-1 (β₂GP-1), funktionellen Derivaten davon und Mischungen davon, in Kombination mit einem pharmazeutisch akzeptablen Träger zur oralen Verabreichung.

4. Eine immunologische und orale toleranzinduzierende Zusammensetzung zur Verhütung und/oder Behandlung des Schlaganfalls durch deren orale Verabreichung, umfassend eine aktive Komponente, gewählt aus der Gruppe bestehend aus modifiziertem niedrigdichtem Lipoprotein, oxidiertem niedrigdichtem Lipoprotein (Ox-LDL), Hitzeschockprotein 60/65 (HSP 60/65), Beta₂-Glucoproteid-1 (β₂GP-1), funktionellen Derivaten davon und Mischungen davon, in Kombination mit einem pharmazeutisch akzeptablen Träger zur oralen Verabreichung.

5. Eine immunologische und orale toleranzinduzierende Zusammensetzung gemäß Anspruch 1, worin besagte aktive Komponente ein modifiziertes niedrigdichtes Lipoprotein ist.

6. Eine immunologische und orale toleranzinduzierende Zusammensetzung gemäß Anspruch 1, worin besagte aktive Komponente oxidiertes niedrigdichtes Lipoprotein (Ox-LDL) ist.

7. Eine immunologische und orale toleranzinduzierende Zusammensetzung gemäß Anspruch 1, worin besagte aktive Komponente ein aktives Derivat oxidierten niedrigdichten Lipoproteins (Ox-LDL) ist.

8. Eine immunologische und orale toleranzinduzierende Zusammensetzung gemäß Anspruch 1, worin besagte aktive Komponente Hitzeschockprotein 60/65 (HSP 60/65) ist.

9. Eine immunologische und orale toleranzinduzierende Zusammensetzung gemäß Anspruch 1, worin besagte aktive Komponente ein aktives Derivat von HSP 60/65 ist.

10. Eine immunologische und orale toleranzinduzierende Zusammensetzung gemäß Anspruch 1, worin besagte aktive Komponente Beta₂-Glucoproteid-1 (β₂GP-1) ist.

11. Eine immmunologische und orale toleranzinduzierende Zusammensetzung gemäß Anspruch 1, worin besagte aktive Komponente ein aktives Derivat von β₂GP-1 ist.

12. Eine immunologische und orale toleranzinduzierende Zusammensetzung gemäß Anspruch 1, worin besagte aktive Komponente ein aktives Derivat von LDL ist, welches aktive Derivat Lysophosphatidylcholin (LPC) ist.

13. Eine immunologische und orale toleranzinduzierende Zusammensetzung gemäß Anspruch 1, worin besagtes LDL Malondialdehyd-LDL (MDA-LDL) ist.

14. Anwendung einer oral verabreichbaren immunologischen und oralen toleranzinduzierenden Zusammensetzung, umfassend eine aktive Komponente, gewählt aus der Gruppe, bestehend aus modifiziertem niedrigdichtem Lipoprotein, oxidiertem niedrigdichtem Lipoprotein (Ox-LDL), Hitzeschockprotein 60/65 (HSP 60/65), Beta₂-Glucoproteid-1 (β₂GP-1), funktionellen Derivaten davon und Mischungen davon, in Kombination mit einem pharmazeutisch akzeptablen Träger zur oralen Verabreichung zur Herstellung eines Medikaments zur Behandlung und/oder Verhütung von Arteriosklerose bei einem Subjekt.

15. Anwendung einer oral verabreichbaren immunologischen und oralen toleranzinduzierenden Zusammensetzung, umfassend eine aktive Komponente, gewählt aus der Gruppe, bestehend aus modifiziertem niedrigdichtem Lipoprotein, oxidiertem niedrigdichtem Lipoprotein (Ox-LDL), Hitzeschockprotein 60/65 (HSP 60/65), Beta₂-Glucoproteid-1 (β₂GP-1), funktionellen Derivaten davon und Mischungen davon, in Kombination mit einem pharmazeutisch akzeptablen Träger zur oralen Verabreichung zur Herstellung eines Medikaments zur Behandlung und/oder Verhütung von Herzanfall bei einem Subjekt.

16. Anwendung einer oral verabreichbaren immunologischen und oralen toleranzinduzierenden Zusammensetzung, umfassend eine aktive Komponente, gewählt aus der Gruppe, bestehend aus modifiziertem niedrigdichtem Lipoprotein, oxidiertem niedrigdichtem Lipoprotein (Ox-LDL), Hitzeschockprotein 60/65 (HSP 60/65), Beta₂-Glucoproteid-1 (β₂GP-1), funktionellen Derivaten davon und Mischungen davon, in Kombination mit einem pharmazeutisch akzeptablen Träger zur oralen Verabreichung zur Herstel-lung eines Medikaments zur Behandlung und/oder Verhütung von Angioplastierestenose bei einem Subjekt.

17. Anwendung einer oral verabreichbaren immunologischen und oralen toleranzinduzierenden Zusammensetzung, umfassend eine aktive Komponente, gewählt aus der Gruppe, bestehend aus modifiziertem niedrigdichtem Lipoprotein, oxidiertem niedrigdichtem Lipoprotein (Ox-LDL), Hitzeschockprotein 60/65 (HSP 60/65), Beta₂-Glucoproteid-1 (β₂GP-1), funktionellen Derivaten davon und Mischungen davon, in Kombination mit einem pharmazeutisch akzeptab-len Träger zur oralen Verabreichung zur Herstellung eines Medikaments zur Behandlung und/oder Verhütung von Schlaganfall bei einem Subjekt.

18. Die Anwendung gemäß einem der Ansprüche 14-17, worin besagte aktive Komponente ein modifiziertes niedrigdichtes Lipoprotein ist.

19. Die Anwendung gemäß einem der Ansprüche 14-17, worin besagte aktive Komponente oxidiertes niedrigdichtes Lipoprotein (Ox-LDL) ist.

20. Die Anwendung gemäß einem der Ansprüche 14-17, worin besagte aktive Komponente ein aktives Derivat von oxidiertem niedrigdichten Lipoprotein (Ox-LDL) ist.

21. Die Anwendung gemäß einem der Ansprüche 14-17, worin besagte aktive Komponente Hitzeschockprotein 60/65 (HSP 60/65) ist.

22. Die Anwendung gemäß einem der Ansprüche 14-17, worin besagte aktive Komponente ein aktives Derivat von Hitzeschockprotein 60/65 (HSP 60/65) ist.

23. Die Anwendung gemäß einem der Ansprüche 14-17, worin besagte aktive Komponente Beta₂-Glucoproteid-1 (β₂GP-1) ist.

24. Die Anwendung gemäß einem der Ansprüche 14-17, worin besagte aktive Komponente ein aktives Derivat von Beta₂-Glucoproteid-1 (β₂GP-1) ist.

25. Die Anwendung gemäß einem der Ansprüche 14-17, worin besagte aktive Komponente ein aktives Derivat von LDL ist, welches aktive Derivat Lisophosphatidylcholin (LPC) ist.

26. Die Anwendung gemäß einem der Ansprüche 14-17, worin besagtes LDL Malondialdehyd-LDL (MDA-LDL) ist.
